# EUROPÄISCHE PATENTANMELDUNG

(11) **EP 1 498 089 A1**
(43) Veröffentlichungstag der Anmeldung: **19.01.2005**
(21) Anmeldenummer: 04020622.9
(22) Anmeldetag: 12.12.2000
(51) Int. Cl.: A61F 2/30, A61F 2/28, A61L 27/00, A61L 27/12, A61B 17/16, A61B 17/32, A61F 2/46

(54) **Präparat für die Reparatur von Knorpel- oder Knorpel/Knochen-Defekten in menschlichen oder tierischen Gelenken**

(30) Priorität: 15.12.1999 CH 229699
(62) Teilanmeldung aus: 00979315.9
(71) Anmelder: Zimmer GmbH, 8404 Winterthur (CH)
(72) Erfinder: Nadler, Daniel, 8442 Hettlingen ZH (CH); Bittmann, Pedro, 8057 Zürich (CH); Akens, Margarete, 8057 Zürich (CH); von Rechenberg, Brigitte, 8903 Birmensdorf (CH); Auer, Jörg, 5600 Lenzburg (CH)
(74) Vertreter: Frei Patent Attorneys

(57) **Zusammenfassung**

Bei der Reparatur von Knorpel- oder Knorpel/Knochen-Defekten in menschlichen oder tierischen Gelenken mit Präparaten, die einen Knochenteil (1), eine Knorpelschicht (2) und im Übergangsbereich zwischen Knorpelschicht (2) und Knochenteil (1) eine subchondrale Knochenplatte (4) oder eine Nachbildung einer derartigen Platte aufweisen, zeigt es sich, dass nach der Implantation der Knochenteil (1) abgebaut und erst nach einem im wesentlichen totalen Abbau durch Reparaturgewebe ersetzt wird. In einer kritischen Phase des Heilungsprozesses befindet sich an der Stelle des implantierten Knochenteils (1) eine mechanisch minderwertige Zyste. Um ein Einsinken der Knorpelschicht (2) in den Zystenraum während der kritischen Phase des Heilungsprozesses zu verhindern, wird vorgeschlagen, das zu implantierende Präparat (10) derart auszugestalten, dass seine Knorpelschicht (2) nach der Implantation auf Bereiche mit voneinander verschiedenem Abbauverhalten abgestützt ist. Dies wird beispielsweise realisiert durch ein Präparat, das einen Kopfteil (11) und einen Fussteil (12) hat, wobei der Kopfteil (11) im wesentlichen aus der Knorpelschicht (2) und der subchondralen Knochenplatte (4) besteht und der Fussteil (12) im wesentlichen dem Knochenteil (1) entspricht und wobei der Kopfteil (11) parallel zur subchondralen Knochenplatte (4) einen grösseren Querschnitt aufweist als der Fussteil (12). Die Knorpelschicht (2) und subchondrale Knochenplatte (4) stützt sich nach der Implantation in einer entsprechenden Öffnung oder Bohrung (20) nicht nur auf dem Knochenteil (1) sondern auch auf nativem Knochengewebe ab, dessen Tragfähigkeit sich während dem Heilungsprozess nicht verändert. Dadurch wird ein Absinken der implantierten Knorpelschicht während dem Heilungsprozess verhindert.

## Beschreibung

Die Erfindung liegt auf dem Gebiete der Medizinaltechnik und betrifft ein Präparat gemäss dem Oberbegriff des unabhängigen Patentanspruchs. Das Präparat dient zur Reparatur von Knorpel- oder Knorpel/Knochen-Defekten in menschlichen oder tierischen Gelenken, das heisst, es dient zur Reparatur von Defekten in der Knorpelschicht, die in Gelenken die Knochenoberfläche abdeckt, oder von Defekten, die diese Knorpelschicht und auch darunterliegendes Knochengewebe betreffen.

Beschädigungen von Gelenkknorpel durch Verletzungen oder durch alters- oder krankheitsbedingte Rückbildung sind insbesondere beim Menschen sehr häufig. Vielfach ist auch das unter dem Gelenkknorpel liegende Knochengewebe durch derartige Beschädigungen in Mitleidenschaft gezogen. Der Schweregrad von Knorpelund Knorpel/Knochen-Defekten wird mit Hilfe der Outerbridge-Skala erfasst, mit den folgenden Kategorien: oberflächliche Ausfransung (ca. 10% aller Fälle), Risse im Knorpel (ca. 28%), Risse bis zum Knochen (ca. 41%), Schäden mit Beteiligung des Knochens (ca. 19%), andere Schäden, wie Osteochondritis dissecans und Gelenkbrüche (ca. 2% aller erfassten Fälle).

Vitales Knorpelgewebe enthält zwar lebende Zellen, durch deren Tätigkeit in der Wachstumsphase die spezifische interzelluläre Knorpelmatrix aufgebaut wird, es ist aber mindestens in ausgewachsenem Zustand kaum durchblutet und besitzt deshalb nach der eigentlichen Wachstumsphase eine nur sehr beschränkte Regenerationsfähigkeit. Das heisst mit anderen Worten, Knorpel- oder Knorpel/Knochen-Defekte, insbesondere derartige Defekte, die eine relativ grosse Knorpeloberfläche betreffen, heilen nicht von selbst und müssen deshalb operativ repariert werden (Mankin HJ: The response of articular cartilage to mechanical injury, Journal of Bone and Joint Surgery (Am) 64A (1982) March: Seiten 460-466).

Für die Reparatur der genannten Defekte wird beispielsweise das Implantieren von Präparaten vorgeschlagen, in denen das zu ersetzende Gewebe bereits vorhanden ist oder möglichst gut vorgebildet ist. Solche Präparate sind zylinderförmig und weisen auf einer Stirnseite eine Knorpelschicht auf. Für die Implantation wird im Bereich des zu reparierenden Defektes eine Sackloch-förmige Öffnung oder Bohrung erzeugt und das Präparat wird derart in die Bohrung eingesetzt, dass die Knorpelschicht des Implantates gegen aussen gerichtet ist. Die Bohrung reicht, unabhängig von der Defekttiefe, bis in gesundes Knochengewebe. Das Präparat weist einen etwas grösseren Durchmesser auf als die Bohrung und dieselbe achsiale Länge. Damit wird erreicht, dass zwischen nativem Gewebe und Implantat eine radiale Spannung entsteht (Pressfit), durch die das Implantat in der Bohrung gehalten wird, und dass die Knorpeloberfläche des Implantates mit der umgebenden nativen Knorpeloberfläche fluchtet. Die Präparate haben je nach Defektgrösse einen Durchmesser von 4 bis 10mm (z.B. 5,4mm für Präparat und 5,3mm für Bohrung) und Längen von ca. 10 bis 20mm.

Für grössere Defekte wird vorgeschlagen, eine Mehrzahl derartiger zylinderförmiger Präparate mosaikartig im defekten Bereich einzusetzen und die Zwischenräume zwischen den Implantaten mit einem geeigneten Material auszufüllen.

Die zylinderförmigen Präparate sind beispielsweise autolog (Autotransplantate). Es wird also beispielsweise für die Reparatur eines Gelenkknorpel-Defektes, wie er an einer stark belasteten Stelle eines Gelenkes entstehen kann, aus einer relativ wenig belasteten Stelle desselben Gelenkes mit einem entsprechenden Hohlbohrer ein geeignetes Gewebestück entnommen und in eine an der defekten Stelle erstellte Bohrung transplantiert (Hangody L et al.: Mosaicplasty for the treatment of articular cartilage defects: application in clinical practice. Orthopedics 1998 Jul,21(7):751-6).

Die zylinderförmigen Präparate können auch von einem geeigneten Spender stammen (homologe Transplantate). Auch entsprechende heterologe oder Xenotransplantate sind bekannt, die vor der Implantation mit einem geeigneten Verfahren, z.B. Photooxidation (wie beschrieben in der Publikation EP-0768332 von Sulzer Innotec), derart behandelt werden, dass eine Immunreaktion nach der Implantation verhindert oder mindestens stark abgeschwächt wird (immunologische Desaktivierung). Solche Implantate werden beispielsweise aus Schultergelenken von geschlachteten Rindern entnommen und haben den Vorteil, dass sie in viel grösseren Mengen zur Verfügung stehen als autologe oder homologe Transplantate und dass sie keine Entnahme-Defekte verursachen, die ihrerseits wieder repariert werden müssen und zu neuen Schwierigkeiten führen können.

In der Publikation WO-97/46665 (Sulzer Orthopedics) wird auch ein entsprechendes Präparat beschrieben, in dem der Knochenteil aus einem Knochenersatzmaterial besteht und in dem die stirnseitige Knorpelschicht beispielsweise ausgehend von autologen Chondrocyten in vitro aufgewachsen wird.

In allen oben genannten Präparaten, die aus natürlichem Gewebe hergestellt werden, gibt es eine natürliche Verbindung oder Verwachsung zwischen der stirnseitigen Knorpelschicht und dem Knochenteil und es gibt einen äussersten Knochenbereich (subchondrale Knochenplatte), in dem das Knochengewebe dichter ist als in anderen Knochenbereichen. Auch die genannten, teilweise künstlichen Implantate weisen die Verwachsung der Knorpelschicht mit dem Knochenteil auf und der künstliche Knochenteil ist vorteilhafterweise mit einer dichteren, das heisst weniger porösen Aussenschicht, die der Knorpelschicht als Unterlage dient, ausgestaltet.

Eine wichtige Funktion der subchondralen Knochenplatte oder einer künstlichen Nachbildung davon ist offenbar die Verhinderung einer vom Knochengewebe ausgehenden Vaskularisierung der Knorpelschicht, die zu einer Verknöcherung dieser Schicht führen würde. Daneben stellt die subchondrale Knochenplatte aber auch einen Bereich dar, der dank seiner höheren Dichte eine höhere mechanische Festigkeit hat als das innere Knochengewebe.

Mit den genannten Präparaten wird versucht, im wesentlichen die folgenden Ziele zu erreichen:
- Der Knochenteil des Präparates soll eine feste Verankerung des Implantates im gesunden Knochengewebe durch Pressfit ermöglichen, derart, dass das Implantat keine weiteren, gesunde Knorpelbereiche beeinflussende Befestigungsmittel benötigt.
- Die Verwachsung von Knorpelschicht und Knochenteil im Präparat soll dem Implantat eine Stabilität geben, derart, dass die Knorpelschicht auch ohne Stilllegung des betroffenen Gelenkes sich nicht ablösen und aus der Defektstelle entfernen kann.
- Die Knorpelschicht des Präparates soll eine derartige mechanische Festigkeit und Elastizität aufweisen, dass die Reparaturstelle schon unmittelbar nach der Implantation voll belastbar ist.
- Die Knorpelschicht soll eine Zone bilden, in der für implantierte Zellen oder für nach der Implantation zuwandernde Zellen geeignete Bedingungen für die Erzeugung und/oder Aufrechterhaltung von funktionsfähigem Knorpelgewebe herrschen. Dies soll auch unterstützt werden durch die subchondrale Knochenplatte, die die Knorpelschicht vom Knochenteil trennt und die hilft, eine vom Knochenteil ausgehende Vaskularisierung der Knorpelschicht zu verhindern.
- Der Knochenteil soll eine Zone darstellen, in der für implantierte Zellen oder für nach der Implantation zuwandernde Zellen geeignete Bedingungen für die Erzeugung und/oder für die Aufrechterhaltung von funktionsfähigem Knochengewebe herrschen.

Neue Versuche, in denen künstlich erzeugte Defekte in Gelenken von Schafen mit Autotransplantaten, mit Homotransplantaten oder mit Heterotransplantaten (aus Rindergewebe) in der eingangs beschriebenen Art repariert wurden, zeigen nun, dass der Heilungsprozess nach der Implantation nicht wie erwartet abläuft.

Es zeigt sich nämlich, dass der Knochenteil der Implantate nicht im nativen Gewebe integriert oder sukzessive durch neues Reparaturgewebe ersetzt wird, sondern dass der Knochenteil des Implantates einen Umwandlungsprozess durchläuft, der im wesentlichen drei aufeinanderfolgende Phasen aufweist. In einem ersten Schritt werden knochenabbauende Zellen (Osteoclasten) stimuliert und der eingesetzte Knochen wird aufgelöst. Diese erste Phase ist bereits nach sechs bis acht Wochen deutlich sichtbar. Mit fortschreitender Implantationsdauer entsteht im Implantat ein Hohlraum (Zyste), der mit Bindegewebe aufgefüllt ist. Diese zweite Phase erreicht einen Höhepunkt nach ca. sechs Monaten. In der dritten und letzten Phase werden knochenbildende Zellen (Osteoblasten) angelockt, welche das Bindegewebe in Knochen umbauen. Der Umbauprozess ist nach ungefähr zwölf Monaten abgeschlossen. Dann ist die neu entstandene Knochenstruktur so gut angepasst, dass die ursprüngliche Grenze zwischen dem Implantat und dem umliegenden Knochengewebe kaum mehr wahrnehmbar ist.

Durch den beschriebenen, dreiphasigen Umwandlungsprozess entsteht in der mittleren Phase, in der die Knorpelschicht des Implantates nicht vom tragfähigen Knochenteil getragen wird sondern von einer mechanisch minderwertigen Zyste, ein hohes Risiko dafür, dass die Knorpelschicht in diese Zyste hineingedrückt wird, wo sie weder ihre mechanische Funktion noch ihre biologische Funktion erfüllen kann und von wo sie während der folgenden Phasen des Heilungsprozesses nicht mehr verschoben werden kann. Durch dieses Risiko werden also die Erfolgschancen der Heilung bedeutend reduziert und es müssen bei einer Verheilung mit schlecht positionierter Knorpelschicht Folgenachteile in Kauf genommen werden.

Erstaunlich an den beschriebenen Befunden ist die Tatsache, dass die Bildung der Zyste an der Stelle des Knochenteils eines Implantates in einer mittleren Phase des Heilungsprozesses nicht nur bei homologen und heterologen Implantaten auftritt, sondern insbesondere auch bei Autotransplantaten. Der anfängliche Abbau des implantierten Knochengewebes scheint also nicht eine Immunreaktion zu sein, in der implantiertes, vitales Material als fremd erkannt und deshalb abgebaut wird. Die Vermutung liegt nahe, dass es sich um eine Reaktion auf implantiertes, totes Material handelt. Dies würde bedeuten, dass durch die Durchtrennung der natürlichen Blutversorgung in einem zwar aus lebensfähigem Gewebe entnommenen Implantat das Knochengewebe stirbt, auch wenn es unmittelbar nach der Entnahme wieder implantiert wird. In jedem Falle baut der Körper den Knochenteil des implantierten Präparates ab, um ihn erst dann wieder neu aufzubauen.

Die Erfindung stellt sich nun die Aufgabe, ein Präparat zur Reparatur von Knorpel-Defekten oder von Knorpel/Knochen-Defekten in menschlichen oder tierischen Gelenken zu schaffen, mit welchem Präparat das oben beschriebene Risiko des Absinkens einer implantierten Knorpelschicht in den Bereich des nativen Knochengewebes verhindert wird. Das erfindungsgemässe Präparat soll in ähnlich einfacher Art hergestellt und implantiert werden können wie die eingangs beschriebenen, bekannten Präparate, die einen Knochenteil und eine mit diesem Knochenteil verwachsene Knorpelschicht aufweisen.

Diese Aufgabe wird gelöst durch das Präparat, wie es im unabhängigen Patentanspruch definiert ist. Die abhängigen Ansprüche definieren bevorzugte Ausführungsformen des erfindungsgemässen Präparates.

Das erfindungsgemässe Präparat zur Reparatur von Knorpel- oder Knorpel/Knochen-Defekten in menschlichen oder tierischen Gelenken basiert auf dem Befund, dass die subchondrale Knochenplatte in der mittleren, kritischen Phase des oben beschriebenen Heilungsprozesses offenbar noch im wesentlichen unverändert vorhanden ist, wenn der Knochenteil vollständig oder grösstenteils durch Bindegewebe ersetzt ist. Dies ist wahrscheinlich darauf zurückzuführen, dass diese subchondrale Knochenplatte wegen ihrer höheren Dichte bedeutend langsamer abgebaut wird als die inneren Bereiche des Knochenteils. Da diese subchondrale Knochenplatte mechanisch genügend stabil ist, kann sie ein Absinken der darauf aufgewachsenen Knorpelschicht in eine darunter entstehende Zyste verhindern, wenn sie nicht nur auf dem Knochenmaterial des implantierten Knochenteils sondern noch zusätzlich auf Material mit anderen Abbau-Eigenschaften derart abgestützt ist, dass sie auch während der kritischen Heilungsphase nicht verschiebbar bleibt. Wenn die subchondrale Knochenplatte des Implantates nach dieser kritischen Phase, das heisst zu einem Zeitpunkt, in dem die Tragfähigkeit des inneren Implantatbereiches wieder hergestellt ist, ebenfalls abgebaut wird, kann dies den Heilungsprozess nicht mehr stark beeinflussen.

Eine Abstützung des implantierten Präparates während der kritischen Heilungsphase lässt sich im wesentlichen auf zwei Arten realisieren.

Einerseits kann das Implantat derart geformt sein, dass die Knorpelschicht und die subchondrale Knochenplatte des Präparates eine grössere Querschnittsfläche haben als der Knochenteil. Ein derartiges Präparat wird in eine zweistufige Bohrung implantiert, derart, dass die subchondrale Knochenplatte des Präparates sich nicht nur auf dem Knochenteil des Implantates sondern auch auf gesundem Knochengewebe neben der für die Reparatur erstellten Bohrung abstützt.

Andererseits können im Knochenteil des Präparates Säulen mit einer reduzierten Abbaubarkeit erstellt werden. Diese Säulen erstrecken sich axial durch den Knochenteil und reichen bis unter die subchondrale Knochenplatte. Die gegenüber den Knochenteilbereichen zwischen den Säulen reduzierte Abbaubarkeit kann realisiert werden durch eine bereichsweise, geeignete chemische Behandlung oder dadurch, dass im Knochenteil des Präparates achsial verlaufende Bohrungen erzeugt und mit einem künstlichen, abbauresistenteren Material gefüllt werden.

Die der Erfindung zugrunde liegenden Befunde sowie beispielhafte Ausführungsformen des erfindungsgemässen Präparates werden im Zusammenhang mit den folgenden Figuren detailliert beschrieben. Dabei zeigen:
- **Figur 1**: ein zylinderförmiges Präparat gemäss dem Stande der Technik zur Reparatur von Knorpel- oder Knorpel/Knochen-Defekten in menschlichen oder tierischen Gelenken;
- **Figuren 2 bis 4**: Gewebeschnitte durch mit einem Präparat gemäss Figur 1 reparierte Knorpel/Knochen-Defekte in einem Schafgelenk zu verschiedenen Zeitpunkten nach der Implantation;
- **Figur 5**: ein Schema der Reparatur eines Knorpel-Defektes mit Hilfe einer bevorzugten Ausführungsform des erfindungsgemässen Präparates (Schnitte entlang der Achse des Präparates bzw. der Bohrung);
- **Figuren 6 und 7**: zwei weitere beispielhafte Ausführungsformen des erfindungsgemässen Präparates im Querschnitt;
- **Figuren 8 und 9**: Schnitte durch einen Knorpel-Defekt (Figur 8) und durch einen Knorpel/Knochen-Defekt (Figur 9), die beide mosaikartig mit einer Mehrzahl von erfindungsgemässen Präparaten gemäss Figur 5 repariert sind.

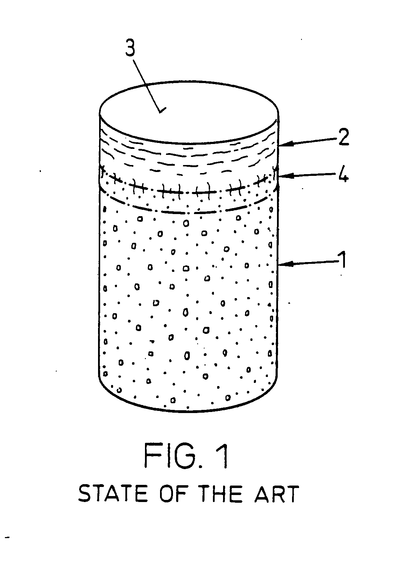

Figur 1 zeigt ein Präparat, wie es nach dem Stande der Technik für die Reparatur von Knorpel- oder Knorpel/Knochen-Defekten an menschlichen und tierischen Gelenken verwendet wird. Das Präparat ist zylinderförmig, vorteilhafterweise mit einem kreisrunden Querschnitt, und weist einen Knochenteil 1 und eine stirnseitig auf dem Knochenteil 1 aufgewachsene Knorpelschicht 2 auf. Die Knorpelschicht 2 bildet eine Knorpeloberfläche 3. Zwischen Knochenteil 1 und Knorpelschicht 2 erstreckt sich eine subschondrale Knochenplatte 4. Die Übergänge von Knochenteil 1 zu subchondraler Knochenplatte 4 und von subchondraler Knochenplatte 4 zu Knorpelschicht 2 sind nicht, wie in der Figur 1 vereinfachend dargestellt, als Linien erkennbar sondern es handelt sich um natürliche eher kontinuierliche Übergänge.

Wie bereits eingangs erwähnt, wird ein Präparat, wie es in der Figur 1 dargestellt ist, vorteilhafterweise aus vitalem Gewebe mit Hilfe eines Hohlbohrers ausgestanzt (Autotransplantate und Homotransplantate) und möglichst sofort implantiert oder es wird aus Gelenken von Schlachttieren (beispielsweise aus Schultergelenken von geschlachteten Rindern) entnommen und vor der Implantation einer Behandlung zur immunologischen Desaktivierung unterzogen.

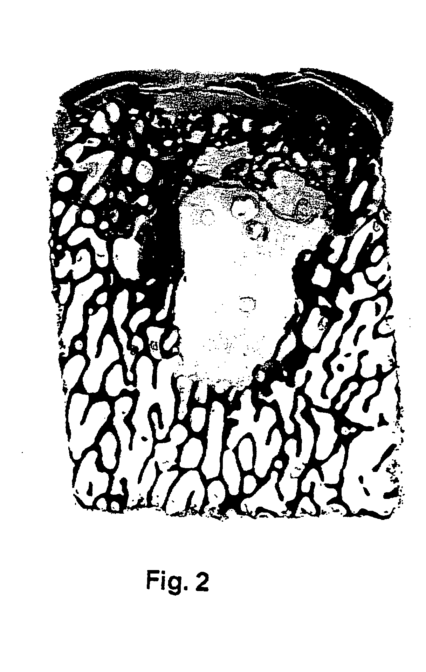

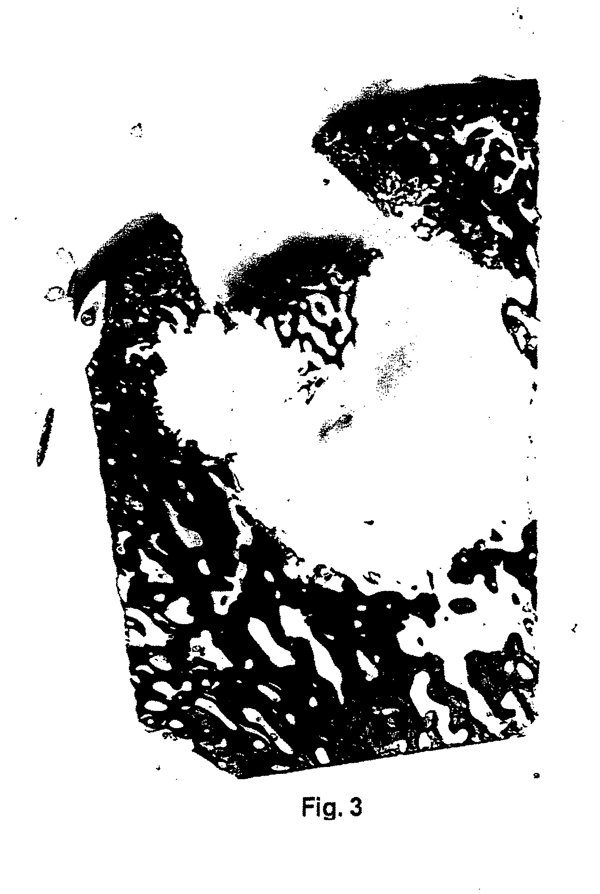

Die Figuren 2 bis 4 illustrieren die weiter oben bereits angeführten Versuche mit Implantaten gemäss Figur 1 in Schafgelenken und das angesprochene Risiko, das Reparaturen mit derartigen Implantaten beinhalten. Es handelt sich um Gewebeschnitte durch Reparaturstellen parallel zur Achse des Implantates, das bei der vorliegenden Vergrösserung von der oben dargestellten Knorpeloberfläche etwa 7 cm in das Knochengewebe hineinragt. Figuren 2 und 3 zeigen Präparate, die sechs Monate nach der Implantation erstellt wurden und in denen an den Reparaturstellen zystenartige Hohlräume im Knochengewebe sichtbar sind. Figur 2 zeigt einen Fall, in dem die Knorpelschicht noch an ihrem ursprünglichen Platz positioniert ist, in Figur 3 ist sie in den Zystenraum abgesunken.

Wie aus den Figuren 2 und 3 ersichtlich ist, ist im kritischen Zeitraum, in dem sich an der Stelle des implantierten Knochenteils ein zystenartiger Hohlraum befindet, die subchondrale Knochenplatte des Implantates noch im Wesentlichen unverändert vorhanden. Dieser Befund lässt sich auf die gegenüber dem inneren Knochengewebe erhöhte Dichte der subchondralen Knochenplatte und auf eine daraus resultierende, reduzierte Abbaubarkeit zurückführen. Die subchondrale Knochenplatte des Implantates hat offensichtlich andere Abbau-Eigenschaften als innere Bereiche des Knochteils desselben Implantates.

Die Versuche wurden mit Autotransplantaten und mit Heterotransplantaten durchgeführt. An 7 behandelten Tieren wurden die Reparaturstellen nach sechs Monaten untersucht und in 10 Fällen (fünf Tiere) wurden in den Zystenhohlraum verschobene Knorpelschichten gefunden, in 4 Fällen (zwei Tiere) waren die Knorpelschichten an Ort und Stelle geblieben. In keinem Fall ging die Knorpelschicht in den Gelenkraum verloren.

Diese Versuchsresultate zeigen, dass offenbar zwischen Zyste und implantierter Knorpelschicht bzw. subchondraler Knochenplatte eine genügende Adhäsion besteht, um die Knorpelschicht daran zu hindern, sich gegen aussen aus der Reparaturstelle zu entfernen, dass aber die Tragfähigkeit der Zyste nicht ausreicht, um die Knorpelschicht daran zu hindern, sich gegen innen zu verschieben.

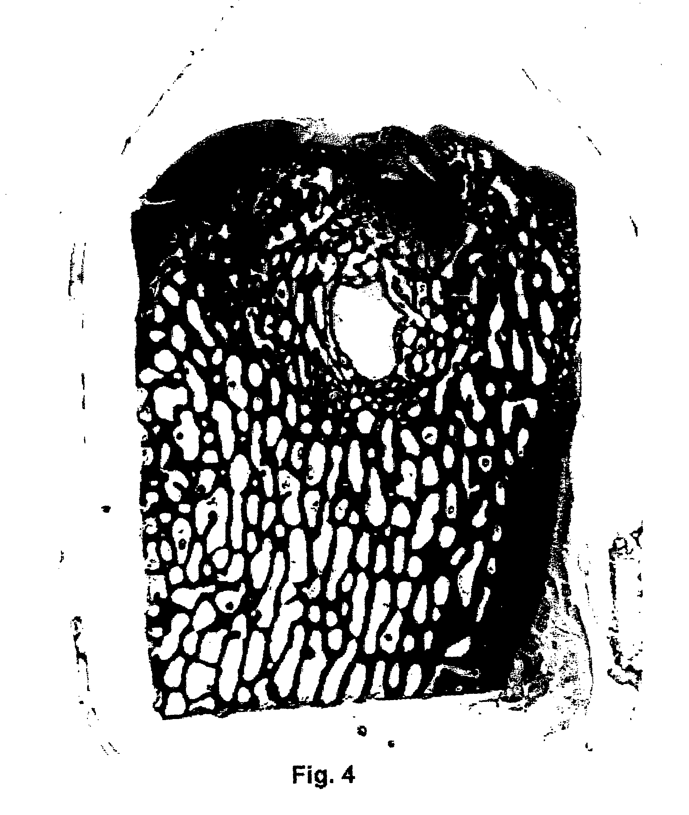

Figur 4 zeigt eine gleiche Reparaturstelle nach zwölf Monaten. Es ist deutlich eine durch das Einsinken der implantierten Knorpelschicht entstandene Unebenheit in der Knorpeloberfläche sichtbar. In der Versuchsreihe wurden Reparaturstellen an sieben behandelten Tieren nach zwölf Monaten untersucht und in zwei Fällen wurden Unebenheiten in der Knorpeloberfläche wie in der Figur 4 gefunden. In den restlichen Fällen war die Knorpeloberfläche im Reparaturbereich eben.

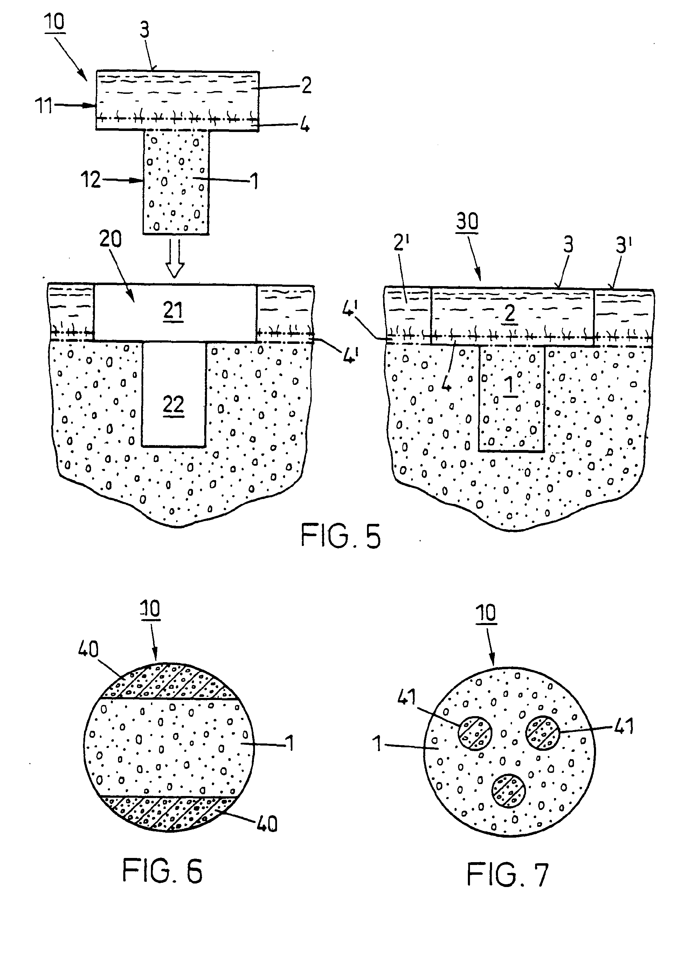

Figur 5 zeigt eine bevorzugte Ausführungsform des erfindungsgemässen Präparates zur Reparatur von Knorpel- oder Knorpel/Knochendefekten in menschlichen oder tierischen Gelenken. Sie zeigt das Präparat 10, die für die Implantation des Präparates 10 zu erstellende Öffnung oder Bohrung 20 und das in die Öffnung eingefügte Präparat, das Implantat 30 (Schnitte entlang der Achse des Präparartes 10 bzw. der Öffnung 20).

Das Präparat 10 hat in derselben Weise wie das Präparat der Figur 1 einen Knochenteil 1, eine stirnseitige Knorpelschicht 2, die eine Knorpeloberfläche 3 bildet, und im Übergangsbereich zwischen Knochenteil 1 und Knorpelschicht 2 eine subchondrale Knochenplatte 4. Das Präparat hat einen Kopfteil 11 mit einem grösseren Querschnitt und einen Fussteil 12 mit einem kleineren Querschnitt. Der Kopfteil 11 umfasst im wesentlichen die Knorpelschicht 2 und die subschondrale Knochenplatte 4, der Fussteil 12 entspricht im wesentlichen dem Knochenteil 1. Der Fussteil 12 hat dabei vorteilhafterweise (aber nicht unbedingt) die Form eines Kreiszylinders oder steilen Kreiskegelstumpfes und der Kopfteil 11 steht allseitig über den Knochenteil 1 vor und ist beispielsweise ebenfalls kreiszylinderförmig.

Autotransplantate und Transplantate von lebenden Donoren haben vorteilhafterweise zylinderförmige Kopfteile, denn derartige Präparate sollten eine nur möglichst kleine Entnahmeöffnung verursachen. Präparate, die aus Gewebe von Schlachttieren (vorteilhafterweise Rinder oder Schweine) hergestellt werden, können ohne weiteres Kopfteile mit verschiedenen Formen von Knorpeloberfläche aufweisen, denn das Material ist in grossen Mengen verfügbar und kann deshalb auch mit Abfall verarbeitet werden. Aber auch in diesem Falle ist es vorteilhaft, den Fussteil derart auszugestalten, dass die für die Implantation zu erstellende Öffnung mit einem einfachen Werkzeug, beispielsweise mit einem Bohrer erstellbar ist.

Ein Präparat 10 mit mindestens im Fussbereich rundem Querschnitt wird beispielsweise aus einem entsprechenden zylinderförmigen Präparat hergestellt dadurch, dass der Knochenteil entsprechend bearbeitet wird. Diese Bearbeitung kann mit einem Werkzeug durchgeführt werden, in dem das zylinderförmige Präparat positionierbar ist und in dem Klingen aktivierbar sind, die in einem vorbestimmten oder einstellbaren Abstand von der Knorpeloberfläche den Querschnitt des Präparates auf ein vorbestimmtes Mass reduzieren.

Die Öffnung oder Bohrung 20, die in einem Defektbereich für die Implantation des Präparates 10 zu erstellen ist, weist einen an den Kopfteil 11 des Präparates 10 angepassten äusseren Bereich 21 auf, der eine Tiefe bis in den Bereich der nativen subchondralen Knochenplatte 4' aufweist, und einen an den Fussteil 12 des Präparates angepassten, inneren Bereich 22, dessen Tiefe sich nach der Form des zu reparierenden Defektes richtet und nach der Länge des zu implantierenden Präparates. Bei der Dimensionierung von Präparat 10 und Bohrung 20 ist für einen Pressfit sowohl im Bereich des Kopfteils als auch im Bereich der Fussteils des Präparates zu sorgen.

Eine Bohrung 20, wie sie in der Figur 5 dargestellt ist, wird beispielsweise erstellt mit einem Werkzeug, das eine Klinge mit einer kreislinienförmigen Schneidekante aufweist und zwei relativ zur Schneidekante axial beschränkt bewegliche Bohrer oder Hohlbohrer. Das Werkzeug wird auf der Defektstelle positioniert und die Klinge bis auf die subchondrale Knochenplatte eingedrückt. Dann wird mit dem ersten Bohrer, der um die Dicke der subchondralen Knochenplatte achsial über die Schneidekante der Klinge hinaus bewegt werden kann und dessen Durchmesser im wesentlichen dem Innendurchmesser der Klinge entspricht, der Äussere Bereich 21 ausgebohrt. Nachher wird mit dem zweiten Bohrer der innere Bereich 22 ausgebohrt, wobei die Bohrtiefe relativ zur Schneidekante oder relativ zur Endposition des ersten Bohrers einstellbar ist.

Figur 5 zeigt auf der rechten Seite auch das Implantat 30, das heisst das in der Bohrung 20 eingesetzte Präparat 10. Das Implantat 30 weist eine mit der nativen Knorpeloberfläche 3' fluchtende Knorpeloberfläche 3 auf und eine mit der nativen subchondralen Knochenplatte 4' in etwa fluchtende subchondrale Knochenplatte 4. Die subchondrale Knochenplatte 4 des Implantates ist offensichtlich abgestützt einerseits auf dem Knochenteil 1 des Implantates und andererseits auf nativem Knochengewebe unmittelbar unter der nativen, subchondralen Knochenplatte 4' oder in deren Bereich. Damit auch der Kopfbereich 11 des Implantates durch einen wirkungsvollen Pressfit in der Bohrung 20 gehalten wird, ist es vorteilhaft, dem äusseren Bereich 21 der Bohrung eine Tiefe zu geben, derart, dass die subchondrale Knochenplatte 4 des Implantates radial nicht nur gegen natives Knorpelgewebe 3' sondern auch gegen natives Knochengewebe (subchondrale Knochenplatte 4') abgestützt ist, wie dies in der Figur 5 dargestellt ist.

Zum Implantieren eines Präparates in einer Bohrung, wie es durch die Figur 5 gezeigt wird, wird beispielsweise ein Werkzeug verwendet, das eine Hülse und einen in der Hülse axial beweglichen Stössel aufweist. Die Hülse hat einen inneren Querschnitt, der dem Querschnitt des Kopfteils des zu implantierenden Präparats entspricht. Der Stössel hat vorteilhafterweise einen etwa gleichen Querschnitt wie dieser Kopfteil; er ist länger als die Hülse und weist einen Kanal auf, der auf der einen Stirnseite des Stössels beginnt und im Bereiche des anderen Stösselendes an eine Saugleitung anschliessbar ist.

Für die Implantation wird die Stirnseite des Stössels mit der Kanalöffnung in die Hülse geschoben und wird mit der erzeugten Saugkraft ein Präparat in die Hülse gezogen. Dann wird die Hülse mit Stössel und dem daran angesaugten Präparat über der vorbereiteten Bohrung positioniert und wird das Präparat mit Hilfe des Stössels und gegebenenfalls eines Hammers in die Bohrung gepresst.

Es zeigt sich, dass durch den Abbau des Knochenteils eines implantierten Präparates auch unmittelbar daran angrenzende Bereiche des nativen Knochengewebes in Mitleidenschaft gezogen werden. Aus diesem Grunde empfiehlt es sich, den Überstand des Kopfteiles auf etwa 1 bis 2 mm zu dimensionieren (z.B. Knochenteil mit Durchmesser ca. 3mm, Kopfteil mit Durchmesser 5 bis 6 mm)

Figuren 6 und 7 zeigen zwei weitere beispielhafte Ausführungsformen des erfindungsgemässen Präparates. Diese basieren auf der Idee, die subchondrale Knochenplatte des Präparates zusätzlich nicht auf nativem Knochengewebe, wie in Figur 5 dargestellt, sondern auf mindestens einer im Knochenteil des Präparates erstellten Säule abzustützen, wobei die Säulen vom restlichen Knochenmaterial des Knochenteils 1 eine verschiedene Abbaubarkeit aufweisen, derart, dass sie in demjenigen Zeitpunkt, in dem das restliche Knochenmaterial des Knochenteils abgebaut ist, die subchondrale Knochenplatte und die damit verwachsene Knorpelschicht tragen und ein Einsinken in den Zystenbereich verhindern können.

Figuren 6 und 7 sind Querschnitte durch Knochenteile 1 von erfindungsgemässen Präparaten. Diese Präparate sind beispielsweise zylinderförmig und weisen im Knochenteil achsial verlaufende Säulen auf, die aus einem Material bestehen, das langsamer abbaubar ist als das Knochenmaterial der Bereiche zwischen den Säulen. Die Säulen sind beispielsweise an der Oberfläche des Knochenteiles angeordnet (Oberflächensäulen 40 in Figur 6) und werden durch eine geeignete Behandlung des Knochenmaterials erzeugt oder sie befinden sich im Innern des Knochenteils (innere Säulen 41 in Figur 7) und werden erzeugt durch Erstellen von Bohrungen und Füllen der Bohrungen mit einem geeigneten Material. In beiden Fällen reichen die Säulen mindestens bis zur subchondralen Knochenplatte.

Als lokale Behandlung des Knochenmaterials für eine reduzierte Abbaubarkeit bietet sich eine Behandlung mit Biphosphonat an ("Biphosphonates in Bone Disease" Herbert Fleisch, The Parthenon Publisching Group, New York und London 1995). Als resorbierbares Material zum Füllen von Bohrungen kann beispielsweise Hydroxylapatitkeramik verwendet werden.

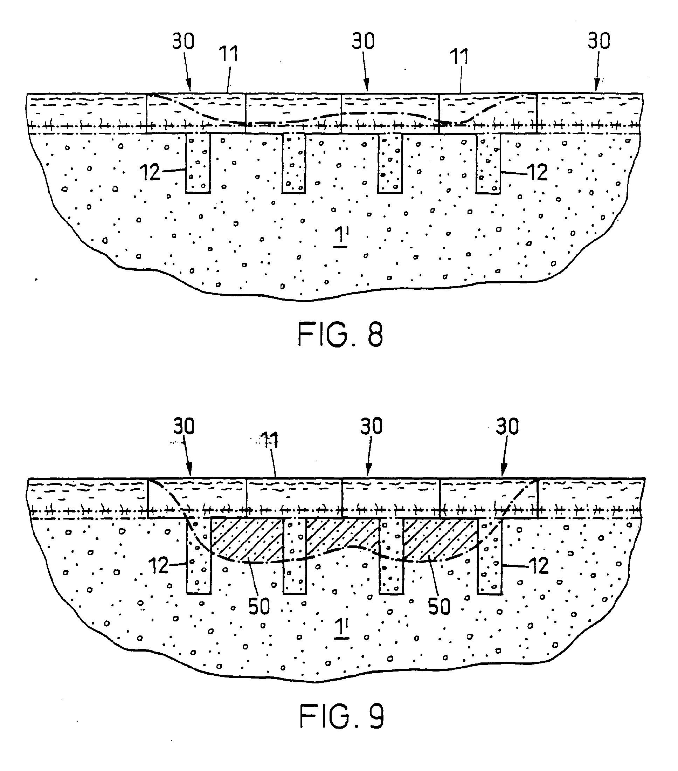

Figuren 8 und 9 zeigen einen Knorpel-Defekt (Figur 8) und einen Knorpel/Knochendefekt (Figur 9) mit Grössen derart, dass sie nicht mit einem einzigen Implantat reparierbar sind. Die Defekte sind mit strichpunktierten Linien angedeutet.

Die Reparatur besteht aus einer mosaikartigen Anordnung von erfindungsgemässen Präparaten, wie sie in der Figur 5 dargestellt sind.

Wie bereits eingangs erwähnt, werden auch mit Implantaten gemäss dem Stande der Technik (Figur 1) derartige mosaikförmige Anordnungen für die Reparatur von grösseren Defekten verwendet. Die zylinderförmigen Präparate werden dabei so nahe beieinander wie möglich implantiert, wobei einerseits Lücken in der Knorpelschicht und andererseits sehr schmale Bereiche von nativem Knochengewebe zwischen den Knochenteilen der Implantate entstehen. Es zeigt sich, dass die Heilungschancen dieser Reparaturen in Randbereichen besser sind als in mittleren Bereichen, und es lässt sich vermuten, dass dies einerseits auf die mangelnde Kompaktheit der neu erstellten Knorpelschicht und auf mangelnde Reparaturfähigkeit des sehr reduzierten nativen Knochengewebes zwischen den Implantaten zurück zu führen ist.

Figur 8 zeigt, dass die Implantate 30 mit Kopfteil 11 auch in einer Mosaik-Reparatur eine Abstützung der Knorpelschicht und der subchondralen Knochenplatte des Implantates auf nativem Knochengewebe 1' erlauben und dadurch einem Absinken der Knorpelschicht in einer kritischen Heilungsphase entgegenwirken. Es ist auch ersichtlich, dass die Bereiche von nativem Knochengewebe 1' zwischen den Fussteilen 12 der Implantate breiter sind als dies bei zylindrischen Implantaten möglich wäre. Dass also auch dadurch die Heilungschancen gegenüber dem Stande der Technik verbessert sind. Und es ist ersichtlich, dass die Knorpeloberfläche über den gesamten Defektbereich im wesentlichen unterbruchslos gestaltet werden kann, wenn die Form der Kopfteile entsprechend (z.B. quadratisch, rechteckig, dreieckig oder sechseckig) gewählt wird.

Figur 9 zeigt einen Knorpel/Knochen-Defekt (mit strichpunktierter Linie angedeutet), der mit einer Mehrzahl von Präparaten gemäss Figur 5 repariert ist. Die Fussteile 12 der Implantate 30 reichen bis in gesundes Knochengewebe 1'. Stellen 50, wo Knochenmaterial fehlt oder beschädigtes Knochenmaterial entfernt wurde, sind mit einem geeigneten Material (beispielsweise Trikalziumphosphat oder hydraulischer Knochenzement) aufgefüllt. Dieses Material ist derart zu wählen, dass es vor oder erst nach den Fussteilen 12 der Implantate 30 abgebaut wird, dass es also in derjenigen, kritischen Phase des Heilungprozesses, in der die Knochenteile der Implantate abgebaut aber noch nicht ersetzt sind, die Knorpelschichten stützen und ein Einsinken der Knorpelschichten verhindern kann. Vorteilhafterweise weist das Füllmaterial eine genügende, mechanische Festigkeit auf, derart, dass es zuerst in den Defekt gefüllt und dann durchbohrt werden kann.

Die für die Reparatur der Figur 8 beschriebenen zusätzlichen Vorteile gelten auch für die Reparatur gemäss Figur 9.

## Patentansprüche

1. Präparat (10) zur Reparatur von Knorpel- oder Knorpel/Knochen-Defekten in menschlichen oder tierischen Gelenken, welches Präparat einen ersten Teil (1) und einen zweiten Teil (4) und eine auf dem zweiten Teil (4) aufgewachsene Knorpelschicht (2) aufweist, **dadurch gekennzeichnet, dass** der erste Teil (1) des Präparates (10) langsamer abbaubar ist als der zweite Teil (4) und dass der erste Teil (1) einen kleineren Querschnitt aufweist als der zweite Teil (4).

2. Präparat nach Anspruch 1, **dadurch gekennzeichnet, dass** der erste Teil (1) kreiszylindrisch ist und dass der zweite Teil (4) stirnseitig an den ersten Teil (1) anschliesst.

3. Präparat nach Anspruch 2, **dadurch gekennzeichnet, dass** der zweite Teil (4) den ersten Teil (1) allseitig überragt.

4. Präparat nach Anspruch 3, **dadurch gekennzeichnet, dass** der zweite Teil (4) mit der aufgewachsenen Knorpelschicht (2) einen zum ersten Teil koaxialen Kreiszylinder bildet.

5. Präparat nach Anspruch 3, **dadurch gekennzeichnet, dass** die auf dem zweiten Teil (4) aufgewachsene Knorpelschicht (2) eine quadratische, rechteckige, dreieckige oder sechseckige Knorpeloberfläche (3) bildet.

6. Präparat nach einem der Ansprüche 1 bis 3, **dadurch gekennzeichnet, dass** der erste Teil (1) und der zweite Teil (4) aus Knochenmaterial oder aus einem Knochenersatzmaterial bestehen und dass das Material des zweiten Teils (4) eine höhere Dichte aufweist als das Material des ersten Teils (1).

7. Präparat nach einem der Ansprüche 1 bis 6, **dadurch gekennzeichnet, dass** es aus autologem Gewebe hergestellt ist.

8. Präparat nach einem der Ansprüche 1 bis 6, **dadurch gekennzeichnet, dass** es aus homologem oder heterologem, immunologisch desaktiviertem Gewebe hergestellt ist.

9. Präparat nach Anspruch 8, **dadurch gekennzeichnet, dass** es aus durch Photooxidation immunologisch desaktiviertem Gewebe hergestellt ist.

10. Präparat nach einem der Ansprüche 8 oder 9, **dadurch gekennzeichnet, dass** es aus aus Schlachttieren entnommenem Gewebe besteht.

11. Präparat nach Anspruch 10, **dadurch gekennzeichnet, dass** es aus Rinder-oder Schweinegelenken entnommen ist.

12. Verwendung von autologem Gewebe oder von homologem oder heterologem, immunologisch desaktiviertem Gewebe zur Herstellung eines Präparates zur Reparatur von Knorpel- oder Knorpel/Knochen-Defekten in menschlichen oder tierischen Gelenken, welches Präparat einen ersten Teil (1) und einen zweiten Teil (4) und eine auf dem zweiten Teil (4) aufgewachsene Knorpelschicht (2) aufweist, wobei der erste Teil (1) des Präparates (10) langsamer abbaubar ist als der zweite Teil (4) und dass der erste Teil (1) einen kleineren Querschnitt aufweist als der zweite Teil (4).
